# EUROPEAN PATENT APPLICATION

(11) **EP 2 228 742 A1**
(43) Date of publication of application: **15.09.2010**
(21) Application number: 09154739.8
(22) Date of filing: 10.03.2009
(51) Int. Cl.: G06F 19/00

(54) **Efficient distribution method for frequency sorting in spectral video analysis of DNA sequences**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

Provided is a method, use, device and computer program product for DNA alignment and sequence analysis of a DNA database comprising a number of sequences of DNA nucleotides. The method, use, device and computer program product has the advantage over the prior art that they allow the ability to distribute for computation both in a scalable manner on available computational resources.

## Description

### FIELD OF THE INVENTION

This invention pertains in general to the field of biology and bioinformatics. More particularly the invention relates to the field of sequence alignment and analysis.

### BACKGROUND OF THE INVENTION

Bioinformatics seeks to organize tremendous volumes of biological data into comprehensible information, which can be used to derive useful knowledge.

A common problem for BLAST and other organization tools known in the art is that the query sequence used as input is limited. If the query sequence length is larger than around a few thousand nucleotides, the search tool will be unacceptably time consuming. Furthermore, with too large query sequences, the accuracy of the search tools diminishes. In order to make existing bioinformatics tools faster and more accurate, the query sequence is usually manually modified and only the data that is deemed to be most relevant is used for searching. This subjective approach is leading to unreliable results because of unacceptable approximations.

DNA spectral analysis offers an approach to systematically tackle the problem of deriving useful information from DNA sequence data. Generally, DNA spectral analysis involves an identification of the occurrences of each nucleotide base in a DNA sequence as an individual digital signal, and transforming each of the four different nucleotide signals into a frequency domain. The magnitude of a frequency component can then be used to reveal how strongly a nucleotide base pattern is repeated at that frequency. A larger magnitude/value usually indicates a stronger presence of the repetition.

Spectral analysis techniques, such as described in WO 2007/105,150, generally represent an improvement over manual DNA pattern analysis techniques, which aim at identifying DNA patterns serving as biological markers related to important biological processes. Traditionally, automatic analyses are performed directly on strings of DNA sequences composed of the four characters A, T, C and G, which represent the four nucleotide bases. However, there are no automatic methods for mining the information available in the spectral video when large numbers of long sequences need to be analyzed. The spectrovideo images quickly require terabytes of data and it is not practical to examine all of them visually. With a spectral image is that for long sequences, such as the entire chromosome 1 of the human genome, with 150 million nucleotides, it is very difficult to spot patterns that appear throughout the genome.

Clustering algorithms currently used for sequence alignment are not suitable for spectral analysis, where the content at individual frequencies needs to be analyzed. Standard clustering methods involve a global distance metric, which in spectral analysis would be applied over all frequencies considered in the spectrogram. While such a method would be able to detect strong patterns in many frequencies, it would screen out strong patterns in individual frequencies.

Therefore, it would advantageous to have a method for the analysis of DNA sequences, which is quick, able to handle large sequences, reliable and cost-effective.

### SUMMARY OF THE INVENTION

Accordingly, the present invention preferably seeks to mitigate, alleviate or eliminate one or more of the above-identified deficiencies in the art and disadvantages singly or in any combination and solves at least the above-mentioned problems by providing a method for DNA sequence analysis based on spectrogram frequencies according to the appended patent claims.

In an aspect, a method for DNA alignment and sequence analysis of a DNA database comprising a number of sequences of DNA nucleotides is provided. Said method comprising dividing the sequences in a DNA database into a number of fragments. Said method further comprises calculating a set of frequency values for each fragment, resulting in a number of DNA spectrogram windows, each comprising the frequency values of a fragment, wherein each frequency value in a DNA spectrogram window corresponds to a frequency and a nucleotide. The method further comprise comparing the frequency values of each fragment with the frequency values of each other fragment, and grouping similar frequency values into sets of frequency values and using the sizes of those sets as input for building a histogram for each frequency, nucleotide and group of windows.

The method has the advantage over the prior art that it has the ability to distribute for computation both the frequency data and the histograms bins in a scalable manner on available computational resources. Also, it is possible to align vast amounts of DNA sequence data in a short time, allowing efficient analysis. Furthermore, the method is scalable with the number of available processing modules and adapts to the dataset size to improve performance and minimize the overhead. It is also possible to extract relevant data quickly, facilitating review of the results by a human observer.

In another aspect, use of a method in designing a test kit for diagnosing genetic diseases is provided.

In yet another aspect, processing unit for DNA sequence analysis based on spectrogram analysis is provided. Said unit is configured to perform the steps according to some embodiments.

In a further aspect, a computer program product is provided. Said computer program product is stored on a computer-readable medium comprising software code adapted to perform the steps of the method according to some embodiments, when executed on a data-processing apparatus.

### BRIEF DESCRIPTION OF THE DRAWINGS

These and other aspects, features and advantages of which the invention is capable of will be apparent and elucidated from the following description of embodiments of the present invention, reference being made to the accompanying drawings, in which
Fig. 1 is a schematic illustration of a method according to an embodiment;
Fig. 2 is a schematic illustration of a method according to another embodiment;
Fig. 3 is a schematic illustration of a method according to yet another embodiment;
Fig. 4 is a schematic illustration of an embodiment of a processing unit;
Fig. 5 is a schematic illustration of an embodiment of a computer program product; and
Fig. 6 is a schematic illustration of an embodiment of the Hierarchy of processing units in distributed Frequency Sorting.

### DETAILED DESCRIPTION OF EMBODIMENTS

Several embodiments of the present invention will be described in more detail below with reference to the accompanying drawings in order for those skilled in the art to be able to carry out the invention. The invention may, however, be embodied in many different forms and should not be construed as limited to the embodiments set forth herein. Rather, these embodiments are provided so that this disclosure will be thorough and complete, and will fully convey the scope of the invention to those skilled in the art. The embodiments do not limit the invention, but the invention is only limited by the appended patent claims. Furthermore, the terminology used in the detailed description of the particular embodiments illustrated in the accompanying drawings is not intended to be limiting of the invention.

An idea according to some embodiments is to use the genomic frequency patterns in biological events, which by systematic spectral analysis may be used in phylogenetic (evolutionary conservation) studies and potentially the discovery of sequence properties in the frequency domain that are not detectable by current string-space linear alignment methods. These new so-called frequency-space sequence properties may be useful classifiers and predictors in genomic biomarker discovery efforts.

Based on the idea above, in an embodiment a distributed frequency sorting method is provided. The frequency sorting method is utilized for detecting all relevant similarities over single frequencies, and not a global similarity over the entire range of frequencies, established according to a global distance metric. The distributed frequency sorting method carries out the alignment of the DNA spectrograms in a scalable and time-efficient manner by making use of distributed computational resources. It will however also be appreciated that the method may work analogously with RNA spectrogram or protein spectrogram applications.

An advantage with combining distributed frequency sorting and spectrovideo is that novel frequency patterns may be discovered in large genomic repositories of sequences. Frequency sorting, compared to previous methods using clustering, has the advantage that it can detect all patterns in individual frequencies. This is particularly important in the case of vast genomic archives, wherever there is large number of sequences and the clustering methods would simply split the pattern across multiple clusters. The algorithm will also detect those patterns detectable by clustering, i.e. combined strong patterns in many frequencies.

However, applying Frequency Sorting to a large dataset is a very data-intensive task, requiring large amounts of computations and being too time consuming to be used in practice for large genomic sequences. In order to be able to apply Frequency Sorting to large datasets and to identify and analyze relevant genomic frequency patterns, the present inventors have designed a method called distributed frequency sorting. In an embodiment, said method allows an efficient distribution of the data and the computation on a large number of processing modules, exploiting the potential of Frequency Sorting for parallelization.

Another advantage of using frequency sorting in our distributed method is that high performance may be achieved and large genomic sequences may be processed, which would not be possible with clustering methods due to their unsuitability for distributed processing.

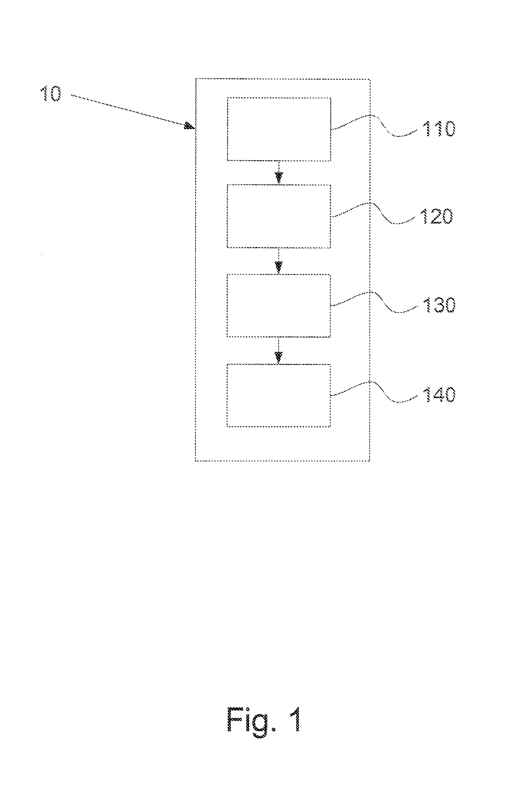

In an embodiment according to Fig. 1, a method 10 for DNA sequence analysis based on spectrogram analysis provided. Said method is using characteristics of the DNA spectrogram dataset and of Frequency Sorting to achieve high performance and low overhead. The method may also be applied to other cases in which many large datasets need to be compared pair-wise, such as for microarray data, for example when microarrays are used to measure a single gene's expression across large patient cohorts.

The method 10 comprises dividing 110 the sequences in a DNA database into a number of fragments. In an embodiment, said DNA database is a genomic energy spectral density database, but several types of DNA databases may be possible. In an embodiment, said dividing 110 results in fragment with constant size. In another embodiment, said dividing 110 results in overlapping fragments. A short-time Fourier transform is calculated for each of the fragments.

Thus, the method 10 further comprises calculating 120 a set of frequency values for each fragment, resulting in a number of DNA spectrogram windows, each comprising the frequency values of a fragment, wherein each frequency value in a DNA spectrogram window corresponds to a frequency and a nucleotide.

The method 10 may also comprise grouping the DNA spectrogram into a DNA spectrovideo comprising frequency windows, according to methods known in the art. This may for example be carried out with a visualization tool allowing the rendering of spectra as video (i.e. spectrovideo), which is implemented to facilitate the analysis of large data sets. An advantage of this is that the large image corresponding to the DNA spectrogram is split into frames, and the user can specify both the number of windows and the number of frequencies per frame. To improve functional interpretation of the spectra, genomic annotation is added to the spectrovideo tool.

Then, the method 10 comprises comparing 130 the frequency values of each fragment with the frequency values of each other fragment. This may result in values in Fourier domain for each nucleotide (A, T, C, G) and for each individual frequency over the entire set of windows. A window represents a set of Fourier values across all frequencies, corresponding to a DNA string of constant size. The spectrum for each window is compared to all the other spectra, per each frequency.

Furthermore, the method 10 comprises grouping 140 similar frequency values into sets of frequency values and using the sizes of those sets as input for building a histogram for each frequency, nucleotide and group of window. In an embodiment the grouping 140 comprises independently counting the numbers of occurrences of similar or identical Fourier values in each column, on one or more processing units, and grouping the identical or similar values. Also the grouping 140 may comprise independently building one histogram per each column, on one or more processing units, wherein each histogram depicts the sizes of the groups of identical or similar values in the column. Furthermore, the grouping 140 may comprise selecting one or more histograms based on a global criterion, applied at one or more processing units. The grouping 140 may also comprise splitting the windows of the DNA spectrogram into groups based on the selection and applying the same reordering to all columns, each group containing one subcolumn for each frequency and each of the four nucleotides. The grouping 140 may further comprise repeating the process independently in each group and in each reordered subcolumn, and for all groups of identical or similar Fourier values by recounting the occurrences of similar or identical Fourier values in each subcolumn. The repeating process may be stopped when a global stopping criterion is reached. Finally, the grouping 140 may comprise generating a final alignment based on the groups of windows. An advantage with this is that there is very little communication required among processing modules working on distinct sets of frequencies.

In an embodiment, the sorting 140 is performed by a single processing unit. In another embodiment, the sorting 140 is performed by multiple processing units. In an embodiment, the number of processing units to participate in the computation of the final aligned DNA spectrogram is chosen and adapted to improve the efficiency and speed of obtaining the final alignment, depending on the size of the DNA spectrogram, as given by numbers of columns and windows.

In an embodiment, when non-identical values are considered similar, the grouping 140 may be done with a binning function. A binning function, i.e. quantization function, is specifying the range of Fourier values to be considered as similar and grouped together. An advantage with this is that similar values may be grouped together to handle small differences in the sequences. For example, genes with similar function in different organisms contain differences in their sequence. However, it may still be desirable to recognize the similarity and align them. The binning function may provide a possibility to distribute the data across computational resources, which will collaboratively provide the computational resources to align the spectrograms. This binning function may be adapted to the dataset. Examples of binning are identity, where identical values are binned together, or values may be truncated to a certain predefined decimal n. However, more complex binning functions may also be defined.

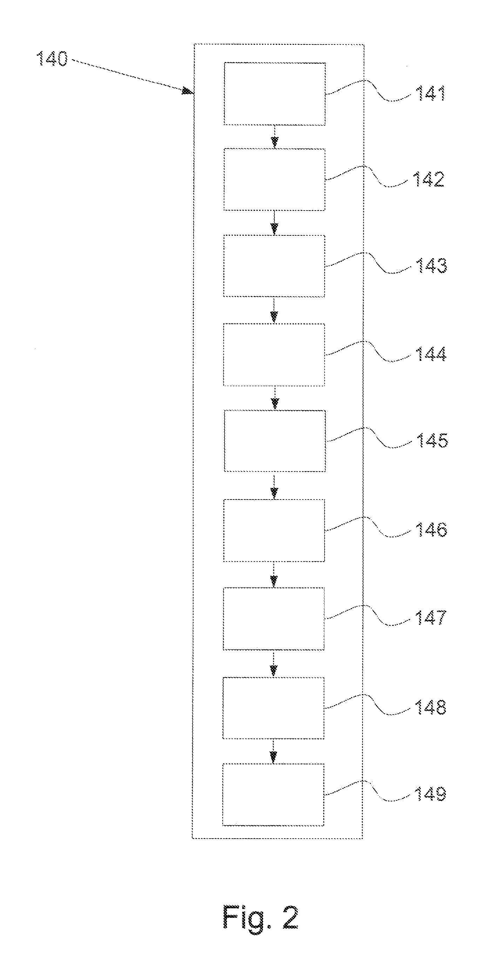

In an embodiment according to Fig. 2, the comparing step 140 is further expanded. The comparing step is performed by calculating 141 a number of Fourier values for each frequency value in the number of DNA spectrogram windows, resulting in a set of Fourier values. Next the comparing step 140 comprises dividing 142 each frequency value in the DNA spectrogram windows into a number of columns, each column comprising said set of Fourier values for one frequency and one nucleotide by creating a number of columns each comprising the set of Fourier values. Next the comparing step 140 comprises counting 143 the numbers of occurrences of similar or identical Fourier values in each column, utilizing a processing unit, and grouping the identical or similar values. Furthermore, the comparing step 140 comprises building 144 one histogram per each column, utilizing a processing unit, wherein each histogram depicts the sizes of the groups of identical or similar values in the column. Next, the comparing step 140 comprises selecting 145 one or more histograms based on a global criterion, applied at a processing unit. Also, the comparing step 140 comprises splitting 146 the DNA spectrogram windows into groups based on the selection and applying the same reordering to all columns, each group containing one subcolumn for each frequency and each of the four nucleotides. Furthermore, the comparing step 140 comprises repeating 147 the process independently in each group and in each reordered subcolumn, and for all groups of identical or similar Fourier values by recounting the occurrences of each similar or identical Fourier values in each subcolumn, or by updating all the counts of the occurrences of similar or identical Fourier values in each subcolumn based the windows that are represented in the new subcolumn after the windows were split, resulting in refined groups of DNA spectrogram windows. Also, the comparing step 140 comprises stopping 148 the repeating step when a global criterion is reached, whereby final refined groups of DNA spectrogram windows is obtained. Finally, aligning 149 of the sequences of DNA nucleotides according to the final refined groups of DNA spectrogram windows, is performed.

A subcolumn will contain the Fourier values in one frequency and one nucleotide across all windows that are part of the group after the split, i.e. the subgroup. We can either count again the occurrences of each identical value (or bin) in the subcolumn, or we can count how many windows that contained the identical value (or bin) are not part of the subgroup at this step compared with the previous step and subtract that value from the count of occurrences for that identical value (or bin).

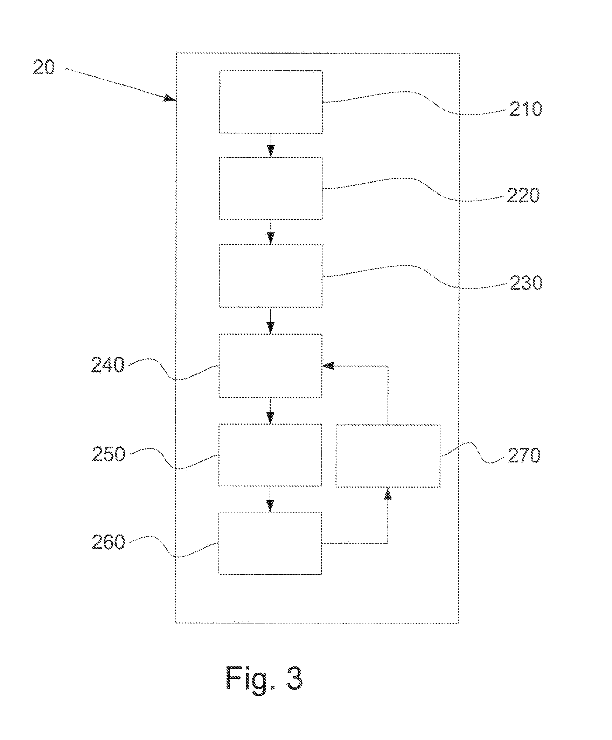

In an embodiment according to Fig. 3, a method 20 is provided for the grouping 140. Said method 20 comprises applying 210 a binning function to the Fourier values, thus assigning them to a bin according to frequency value similarity. Furthermore, the method 20 comprises distributing 220 Fourier values over the total number of available processing units, P, per frequency and nucleotide, thus giving one or several columns to each processing unit, where each column is comprising the Fourier values corresponding to a single frequency and a nucleotide across all windows. The method 20 also comprises splitting 230 the computation of bins for assigned frequencies and nucleotides among several processing units. The method 20 further comprises choosing 240 winning bins, for each processing node, among the assigned frequencies based on which to split the windows on the DNA spectrogram and to reorder them into groups. Furthermore, the method 20 comprises comparing 250 values with other processing units and chose the global winning bin. The method 20 also comprises splitting 260 the DNA spectrogram into groups of windows and reordering the windows according to selected bins, such as the largest bin, in one or more frequencies, by shuffling the windows in the group to have all the windows containing the frequency values that belong to the winning bins in a consecutive order. Choosing 240 winning bins, comparing 250 values and splitting 260 the DNA spectrogram for each processing node is iterated 270. Said iteration 270 may be until a stopping criterion, such as when no reordering is necessary, or when each group at most contains two windows.

Next, the method according to some embodiments is selecting one or more histogram bins for each frequency, according to a chosen strategy. Such strategy may be bin size. When the largest bin is selected, it provides the largest number of sequences that share a similar value according to the binning function, in that specific frequency, for one of the nucleotides. Thus, the method comprises finding a single histogram for each frequency.

The method further comprises selecting a frequency, for example the one corresponding to the largest value in all histogram bins across all frequencies and grouping the sequences contributing to that histogram together. In this way, the whole domain of sequences is split into the group of sequences sharing a similarity in that frequency and the rest, obtaining two groups, and a specific selection and processing strategy is applied on each of the two groups. Next, the method comprises building histograms of the values again or updating the computed histograms bins to reflect the split into groups; the longest histogram is selected, and according to that histogram the domain is split into two groups again. The iterations stop when the size of the longest histogram is below a predefined threshold, when the user-defined number of long patterns to be extracted was reached, or when each of the two groups contains a single sequence.

According to frequency sorting, the bin sizes are computed independently, during each iteration, for each frequency and nucleotide. The method may comprise comparing bin values across all frequencies and nucleotides, and based on that the domain of windows is then reordered and split.

For large data sets with many frequencies and/or windows, computation of the bin sizes to generate histograms for each frequency is a very time-consuming task. However, when sorting the histograms, they are built independently for each frequency. This makes it efficient to split y the Fourier values per each frequency based on which of the histograms is computed among several processing modules and execute them in parallel, on a parallel or distributed system. An advantage with this is that there is very little communication required among processing modules working on distinct sets of frequencies. In an embodiment, the method comprises computation of histograms of Fourier values, split per frequency (histogram) and distributed to several processing modules. Thus, each available processing module is receiving a set of frequencies (one or more) for which to compute the bin sizes and build the histogram. At the end, the method comprises comparing the results among the processing modules and a making a decision concerning the split of the domain. When a processing module has the task to build several histograms, each corresponding to a distinct frequency, it may choose one of them, e.g. the largest in one embodiment, to compare with the histograms selected by the other processing modules. The comparison can be implemented by sending all relevant values to all other processing units, or specializing a processing unit to carry out the comparison of the histograms selected by all processing units, or of all histograms in the domain if the selection step at the level of worker processing units is not carried out. Based on the comparison result (that for example selects the largest histogram across all frequencies) all processing units will apply the split decided. When a single processing unit is responsible for the histogram comparison step, that unit will notify the others of the split. In the embodiment in which the largest histogram is selected, the split will group together all the windows contributing to the largest histogram. When the longest histogram size is shared by several histograms, one of them can be selected according to a criterion. In an embodiment, such criterion is a histogram computed by the processing unit with the smaller index.

This way, the processing modules may work independently on parts of the domain and the entire data set may be processed faster, with little distribution and communication overhead. Depending on the number of frequencies, which gives the number of columns, the number of rows, and on the available processing units, the number of histograms per processing unit may be varied to improve the performance-resource utilization ratio. Various approaches to assign histograms to processing units may be considered, such as round robin, which means that all the columns are distributed among the available processing unit in a specific order, in an iterative fashion until all the columns are assigned, pool of work, which means that each processing unit claims one column at a time for which it computes a histogram, then iteratively claims a new column until all histograms are built, differentiated per processing unit, such as processing units linked by faster connections, which may collaborate more efficiently for building a histogram, performance features , which considers other requirements when assigning work, such as that slower processing units receive lower workload, etc.

In an embodiment, the method is distributing the computation of each histogram on several processing units. Each processing unit is responsible for the computation at each step of one or more bin sizes, i.e. the number of occurrences of values belonging to that bin. These results are combined into a histogram and compared to the other histograms. The distribution of bins is useful when the number of processing units available is larger than that of histograms to be built in each iteration, and the number of rows is very large, so the computation of each histogram, i.e. all bins for one frequency and all four nucleotides, is still considered too time consuming to be handled in its entirety by a single processing unit.

For example, if the window size is 600, this yields 301 relevant frequencies, and 1204 columns (for all 4 nucleotides and all frequencies). A column contains Fourier values for a frequency across windows. When the number of available processing nodes is lower than 1204, a distribution of only columns is suitable. Having access to more nodes may be beneficial when the execution time for processing each column is still large and a speed-up is desired, such as when processing many genomes of different organisms. In that case, when for example an additional 1204 nodes are available, the workload of building bins could be split, and for each column of values there will be two processing units cooperating. The next run, different numbers of processing nodes, chosen from a different window size, may be used for processing columns and bins. Processing nodes are assigned to compute columns but may also participate to the processing of bins in such way that the load is balanced.

The bins may be computed at each step by scanning the entire column corresponding to a frequency and to each of the four nucleotides, or they may be updated in each iteration based on the current split of the domain. In this case the list of windows contributing to the bin at the previous step need to be preserved together with the previous size of the bin. In the first iteration all the windows are part of that list. During each iteration, and for each bin, the update of the bin size is done by comparing the list of windows contributing to the current bin, to the list of windows in the current domain after the split of the column. Next, the list of windows is updated: All windows that contributed to the bin in the previous iteration but due to the split are no longer part of the domain in the current iteration are removed from the list specifying the windows contributing to the bin. Subsequently, the size of the bin is updated (i.e. decreased) accordingly by subtracting from its current size the number of windows removed from the list of windows.

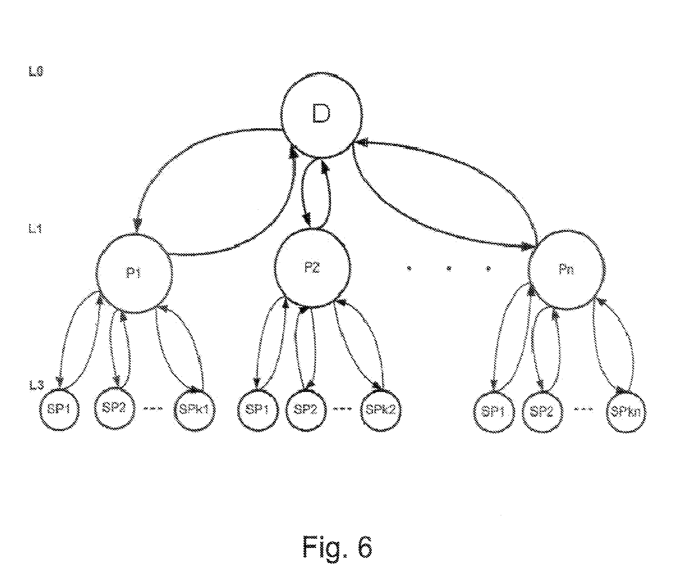

To improve the performance of Frequency Sorting and the utilization of the available resources, the distribution of the histograms and bins among processing units is adaptive, and may be modeled by a tree with three levels, according to Fig. 6. For small datasets, all histograms may be computed on a single processing unit. At the next level, individual histograms are assigned to distinct processing units. On the third level, one or several bins are assigned to each processing unit by a corresponding processing unit on the second level of the tree. The load is dynamically balanced (the bins are split) among the processing unit on the second level and its sub-units on the third level. This distribution method allows efficient, flexible and scalable sorting and analysis of increasing sequence sizes, including entire genomes. It also scales well with the variation of the window size, which yields the number of frequencies in the domain. Large datasets could still be analyzed with a small window to detect small patterns, without decreasing the performance as the method also scales on bins and allows distribution of bins among processing units.

As with DNA spectrograms the window size influences the size of the patterns to be visualized and the dataset needs to be analyzed for several window sizes. The number of frequencies increases with the window size, while the number of bins decreases. This yields that the numbers of processing nodes at the second and third level also vary with the window size.

Assuming that all processing units are equivalent and have the same performance, only columns corresponding to Fourier values for a frequency and a nucleotide will be distributed, when the total number of processing units available is smaller or equal to the number of columns: *Nₚ* ≤ (*w*/2+1)^{*4}, where w is the window size of the short-time Fourier transform (STFT), and Nₚ is the number of processing units available.

To speed-up the alignment of the DNA spectrogram, the split on bins can be considered when *Nₚ*>(*w*/2+1)^{*4} or when the performance of the available processing units is different. Several slower processing units can share the computation of the bins in a column.

In an embodiment Frequency Sorting and the Top Down Hierarchical Sorting (TDHS) algorithm may be combined. A tree structure is used to enable search in the domains of windows and to enable visualization of the final set of sorted windows together with information about how the windows group together according to the Frequency Sorting method and the algorithm used (TDHS).

With TDHS first the longest pattern (i.e. the one that corresponds to the largest histogram bin) in any of the frequencies is computed. The largest histogram bin in a frequency provides the largest number of windows that share a similar value according to the binning function in that specific frequency for one of the nucleotides. Once the largest value in all histogram bins across all frequencies, for each frequency there is a single histogram, is found, the frequency of the largest histogram bin is selected and the windows contributing to the longest histogram bin, i.e. longest pattern, are grouped together.

Based on the longest pattern, TDHS splits the domain of windows into those containing the longest pattern and the rest. The whole domain of windows is split this way into the group sharing a similarity in that frequency and the rest, obtaining two groups.

In the next iterations, histograms of the values are built again or the computed histogram bins are updated to reflect the split into groups. In each of the two clusters, the second) longest histogram bin is selected and the domain is again split into two clusters. The iterations stop when the size of the longest histogram is below a predefined threshold, when the user-defined number of long patterns to be extracted is reached, or when each of the two groups contains a single sequence. In the end a hierarchy of patterns is obtained.

Each level represents an iteration, and the entire tree shows how the domain of windows of Fourier values was iteratively sorted and split, until the final sorted spectrogram was obtained. Each node corresponds to a set of windows split from the domain at the parent node and grouped together at that iteration.

The root node (iteration 0) corresponds to the entire domain of windows to be sorted, including all sequences (rows of Fourier values, or binned Fourier values) to be sorted, corresponding to all frequencies and all four nucleotides.

In an embodiment according to Fig. 6 a tree of processing nodes participating to the execution of the alignment algorithm (Frequency Sorting with TDHS) is provided. The distributor node on level L0, D, is responsible for distributing the dataset among processing units on levels L1 and L3, P and SP, respectively, choosing the winning pattern () at each step, and compiling the alignment, i.e. reordering of the rows. A pattern is a set of Fourier values in a same frequency contributing to the same bin. In another embodiment, the nodes responsible for computing columns could choose the winning pattern collaboratively.

The processing units P₁ ... Pₙ receive from the distributor the columns to compute. To minimize the overhead of data transfer, the columns are assigned at the beginning of the execution and will not change.

Repeatedly, until a stopping criterion is reached, each Pi builds the histograms and chooses the winning bin for each of its assigned columns, then collaboratively or though the distributor, P₁ ... Pₙ choose a global winning bin and reorder or split their dataset accordingly. The decision will have to be recorded at the distributor as well.

The processing units SP₁ ... SPₖᵢ, i=1..n are responsible for updating the bins assigned to them by the Pᵢ processing units. They repeatedly update the sizes of the bins based on the new reordering or split of the domain sent to them by the Pi units. Figure 3 also describes the main execution steps of the distributed frequency sorting method.

In an embodiment, the method according to some embodiments may be used in designing a test kit for diagnosing genetic diseases.

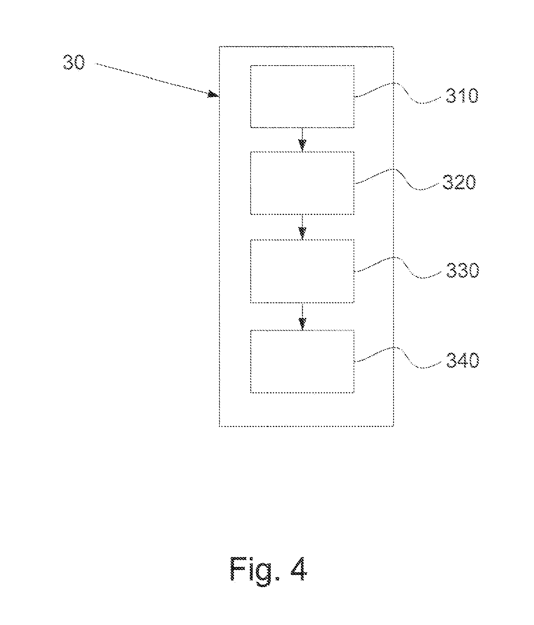

In an embodiment according to Fig. 4, a processing unit 30 for DNA sequence analysis based on spectrogram analysis is provided. Said unit 30 is configured to divide 310 the sequences in a DNA database into a number of fragments, wherein each fragment has constant size, and wherein at least two fragments overlap. Said unit 30 is also configured to calculate 320 a set of frequency values for each fragment, resulting in a DNA spectrogram comprising the frequency values of each fragment. Furthermore, the unit 30 is configured to compare 330 the set of frequency values of each fragment with the set of frequency values of each other fragment, and grouping similar fragments into histograms. The unit 30 is also configured to sort 340 the DNA database based on the fragments of each histogram.

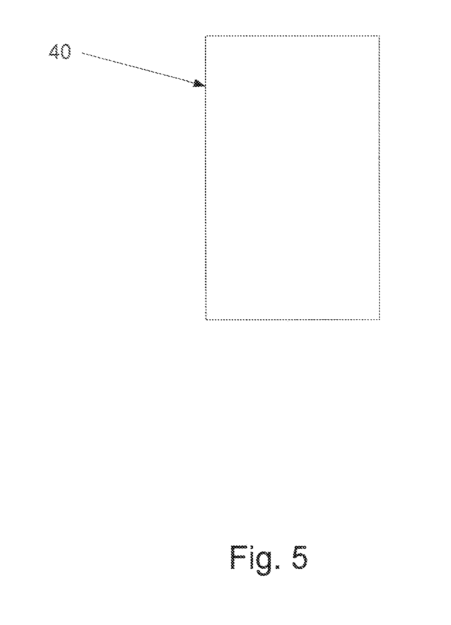

In an embodiment according to Fig. 5, a computer program product 40 is provided. Said computer program product is stored on a computer-readable medium comprising software code adapted to perform the steps of the method according to some embodiments when executed on a data-processing apparatus.

The invention may be implemented in any suitable form including hardware, software, firmware or any combination of these. However, preferably, the invention is implemented as computer software running on one or more data processors and/or digital signal processors. The elements and components of an embodiment of the invention may be physically, functionally and logically implemented in any suitable way. Indeed, the functionality may be implemented in a single unit, in a plurality of units or as part of other functional units. As such, the invention may be implemented in a single unit, or may be physically and functionally distributed between different units and processors.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

In the claims, the term "comprises/comprising" does not exclude the presence of other elements or steps. Furthermore, although individually listed, a plurality of means, elements or method steps may be implemented by e.g. a single unit or processor. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A method (10) for DNA alignment and sequence analysis of a DNA database comprising a number of sequences of DNA nucleotides, comprising the steps:
dividing (110) the sequences in a DNA database into a number of fragments;
calculating (120) a set of frequency values for each fragment, resulting in a number of DNA spectrogram windows, each comprising the frequency values of a fragment, wherein each frequency value in a DNA spectrogram window corresponds to a frequency and a nucleotide;
comparing (130) the frequency values of each fragment with the frequency values of each other fragment, and
grouping (140) similar frequency values into sets of frequency values and using the sizes of those sets as input for building a histogram for each frequency, nucleotide and group of windows.

2. The method according to claim 1, wherein said comparing step (140) is performed by:
calculating (141) a number of Fourier values for each frequency value in the number of DNA spectrogram windows, resulting in a set of Fourier values;
dividing (142) each frequency value in the DNA spectrogram windows into a number of columns, each column comprising said set of Fourier values for one frequency and one nucleotide by creating a number of columns each comprising the set of Fourier values;
counting (143) the numbers of occurrences of similar or identical Fourier values in each column, utilizing a processing unit, and grouping the identical or similar values;
building (144) one histogram per each column, utilizing a processing unit, wherein each histogram depicts the sizes of the groups of identical or similar values in the column;
selecting (145) one or more histograms based on a global criterion, applied at a processing unit;
splitting (146) the DNA spectrogram windows into groups based on the selection and applying the same reordering to all columns, each group containing one subcolumn for each frequency and each of the four nucleotides;
repeating (147) the process independently in each group and in each reordered subcolumn, and for all groups of identical or similar Fourier values by recounting the occurrences of each similar or identical Fourier values in each subcolumn, or by updating all the counts of the occurrences of similar or identical Fourier values in each subcolumn based the windows that are represented in the new subcolumn after the windows were split, resulting in refined groups of DNA spectrogram windows;
stopping (148) the repeating step when a global criterion is reached, whereby final refined groups of DNA spectrogram windows is obtained; and
aligning (149) the sequences of DNA nucleotides according to the final refined groups of DNA spectrogram windows.

3. Method according to claim 1, wherein the fragments resulting from the division (110) have constant size.

4. Method according to claim 1, wherein the fragments resulting from the division (110) overlap.

5. Method according to any of claims 1-4, wherein the grouping (140) is performed by a single processing unit.

6. Method according to any of claims 1-5, wherein the grouping (140) is performed by multiple processing units.

7. Method according to claim 6, wherein the grouping (140) is performed by:
applying (210) a binning function to the Fourier values, thus assigning them to a bin according to frequency value similarity;
distributing (220) Fourier values over the total number of available processing units, P, per frequency and nucleotide, thus giving one or several columns to each processing unit, where each column is comprising the Fourier values corresponding to a single frequency and a nucleotide across all windows;
splitting (230) the computation of bins for assigned frequencies and nucleotides among several processing units;
choosing (240) winning bins, for each processing node, among the assigned frequencies based on which to split the windows on the DNA spectrogram and to reorder them into groups;
comparing (250) values with other processing units and choosing the global winning bin;
splitting (260) the DNA spectrogram into groups of windows and reordering the windows according to selected bins in one or more frequencies, by shuffling the windows in the group to have all the windows containing the frequency values that belong to the winning bins in a consecutive order; and
iterating (270) choosing (240) winning bins, comparing (250) values and splitting (260) the DNA spectrogram for each processing node.

8. Method according to claim 6, wherein depending on the size of the DNA spectrogram, as given by numbers of columns and windows, the number of processing units to participate in the computation of the final aligned DNA spectrogram is chosen and adapted to improve the efficiency and speed of obtaining the final alignment.

9. Method according to claim 1, wherein said DNA database is a genomic energy spectral density database.

10. Method according to claim 1, wherein said DNA spectrogram is a micro array.

11. Method according to claim 1, wherein said DNA spectrogram consists of several microarrays.

12. Use of the method according to any one of claims 1 to 11 in designing a test kit for diagnosing genetic diseases.

13. A processing unit (30) for DNA sequence analysis based on spectrogram analysis, configured to:
dividing (310) the sequences in a DNA database into a number of fragments;
calculating (320) a set of frequency values for each fragment, resulting in a number of DNA spectrogram windows, each comprising the frequency values of a fragment, wherein each frequency value in a DNA spectrogram window corresponds to a frequency and a nucleotide;
comparing (330) the frequency values of each fragment with the frequency values of each other fragment; and
grouping (340) similar frequency values into sets of frequency values and using the sizes of those sets as input for building a histogram for each frequency, nucleotide and group of windows.

14. A computer program product (40) stored on a computer-readable medium comprising software code adapted to perform the steps of the method according to any of claims 1 to 11, when executed on a data-processing apparatus.
